(19) Europäisches Patentamt
European Patent Office
Office européen des brevets

(11) Veröffentlichungsnummer: **0 571 857 A1**

# (12) EUROPÄISCHE PATENTANMELDUNG

(21) Anmeldenummer: **93107998.2**

(22) Anmeldetag: **17.05.93**

(51) Int. Cl.[5]: **C07D 213/42**, C07D 295/195, C07D 413/12, A01N 47/24, A01N 47/34

(30) Priorität: **29.05.92 DE 4217723**

(43) Veröffentlichungstag der Anmeldung:
**01.12.93 Patentblatt 93/48**

(84) Benannte Vertragsstaaten:
**BE CH DE ES FR GB IT LI NL**

(71) Anmelder: **BAYER AG**
**D-51368 Leverkusen(DE)**

(72) Erfinder: **Heuer, Lutz, Dr.**
**Scheibler Strasse 83**
**W-4150 Krefeld(DE)**
Erfinder: **Kugler, Martin, Dr.**
**Am Kloster 47**
**W-5653 Leichlingen(DE)**
Erfinder: **Paulus, Wilfried, Dr.**
**Deswatinesstrasse 40**
**W-4150 Krefeld(DE)**
Erfinder: **Lorentzen, Jens-Peter, Dr.**
**An der Ruthen 2**
**W-5000 Köln 80(DE)**
Erfinder: **Dehne, Heinz-Wilhelm, dr.**
**Krischerstrasse 81**
**W-4019 Monheim(DE)**
Erfinder: **Erdelen, Christoph, Dr.**
**Unterbüscherhof 22**
**W-5653 Leichlingen(DE)**

(54) **Thiosemicarbazide.**

(57) Beschrieben wird die Verwendung von Thiosemicarbaziden der Formel (I)

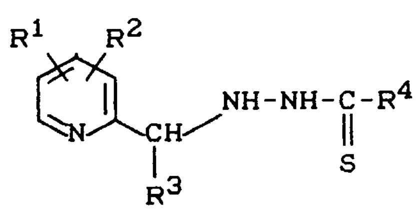

(I)

in welcher $R^1$, $R^2$, $R^3$ und $R^4$ die in der Beschreibung angegebene Bedeutung haben zum Schutz technischer Materialien, sowie neue Thiosemicarbazide, deren Herstellung und deren Verwendung als antimikrobielle Mittel im Materialschutz und Pflanzenschutz.

EP 0 571 857 A1

Die Anmeldung betrifft die Verwendung von Thiosemicarbaziden zum Schutz technischer Materialien, sowie neue Thiosemicarbazide, deren Herstellung sowie deren Verwendung als antimikrobielle Mittel im Materialschutz und Pflanzenschutz.

Thiosemicarbazide und deren fungizide Verwendung im Pflanzenschutz werden in WO-8 504 333 beschrieben.

Die Wirksamkeit dieser vorbekannten Verbindungen ist jedoch insbesondere bei niedrigen Aufwandmengen und Konzentrationen nicht in allen Anwendungsgebieten zufriedenstellend. Desweiteren ist deren Verwendung zum Schutz technischer Materialien noch nicht bekannt.

Es wurde nun überraschenderweise gefunden, daß das Wirkspektrum und die Wirkaktivität dieser Verbindungen deren Einsatz auch zum Schutz technischer Materialien ermöglicht.

Gegenstand der Anmeldung ist daher die Verwendung der Verbindungen der Formel (I)

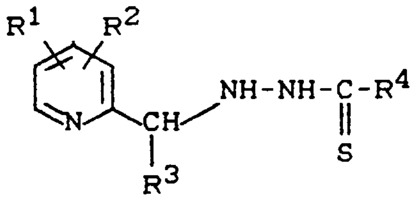

(I)

in welcher

- $R^1$ und $R^2$ unabhängig voneinander jeweils für Wasserstoff, Alkyl, Alkoxy, Thioalkyl, Halogen, Halogenalkyl, Halogenalkoxy oder Halogenthioalkyl stehen oder zusammen einen Benzol- oder Cycloalkylring bilden,
- $R^3$ für Wasserstoff, Alkyl, Cycloalkyl, Aryl, Aralkyl, Hetaryl oder Hetaralkyl steht und
- $R^4$ für die Gruppen $SR^5$, $NR^6R^7$ oder $OR^5$ steht, wobei
- $R^5$ für Alkyl, Cycloalkyl, Cycloalkenyl, Aralkyl oder Aryl steht und
- $R^6$ und $R^7$ unabhängig voneinander jeweils für Wasserstoff, $R^5$ oder die Gruppe $-CH_2-R^3$ stehen, oder zusammen mit dem Stickstoffatom einen gesättigten bis ungesättigten, gegebenenfalls substituierten Heterocyclus bilden,

sowie deren Metallsalzkomplexe und Säureadditions-Salze zum Schutz von technischen Materialien.

Alkyl, Alkoxy und Thioalkyl stehen hierbei und im folgenden für geradkettige oder verzweigte Reste mit vorzugsweise 1 bis 18, insbesondere 1 bis 10, bevorzugt 1 bis 6, besonders bevorzugt 1 bis 4 Kohlenstoffatomen wie Methyl, Ethyl, i-, n-Propyl, i-, n-, s-, tert.-Butyl, Methoxy, Ethoxy, i-, n-Propyloxy, i-, n-, s-, tert.-Butyloxy, Methylthio, Ethylthio, i-, n-Propylthio und i-, n-, s-, t-Butylthio.

Halogen steht hierbei und im folgenden, alleine oder in zusammengesetzten Resten, für Fluor, Chlor, Brom und Iod insbesondere Fluor und Chlor, wobei die Halogenalkyl-, -alkylthio und -alkoxyreste vorzugsweise jeweils 1 bis 9, inbesondere 1 bis 3 gleiche oder verschiedene Halogenatome tragen.

Cycloalkyl und Cycloalkenyl steht hierbei und im folgenden vorzugsweise für gegebenenfalls durch Halogen, Alkyl und/oder Alkoxy substituiertes Cyclopropyl, Cyclobutyl, Cyclopentyl, Cyclohexyl, Cycloheptyl und Cyclooctyl beziehungsweise deren teilweise ungesättigte Analoge.

Aryl und Aralkyl steht hierbei und im folgenden vorzugsweise für gegebenenfalls durch Halogen, Alkyl, Alkoxy und/oder Thioalkyl substituiertes Phenyl, Naphthyl, Benzyl und Phenylethyl.

Hetaryl und Hetaralkyl steht hierbei und im folgenden vorzugsweise für 2-, 3- oder 4-Pyridyl, Pyrimidyl, Thienyl, Furfuryl oder Pyrryl beziehungsweise deren über Alkyl, vorzugsweise die Methylen-Gruppe gebundene Analoge.

Bevorzugt werden die Verbindungen der Formel (I) verwendet, in welcher

- $R^1$ und $R^2$ unabhängig voneinander für Wasserstoff, Halogen, $C_1$-$C_6$-Alkyl, -Alkoxy, -Thioalkyl, -Halogenalkyl, -Halogenalkoxy oder -Halogenthioalkyl stehen oder zusammen einem Benzol-, Cyclopentan- oder Cyclohexanring bilden
- $R^3$ für Wasserstoff, $C_1$-$C_{10}$-Alkyl, $C_3$-$C_8$-Cycloalkyl, Phenyl, Naphthyl, Benzyl, Pyridyl, Pyrimidyl, Thienyl, Furfuryl oder Pyrryl steht und
- $R^4$ für die Gruppe $SR^5$, $NR^6R^7$ oder $OR^5$ steht, wobei
- $R^5$ für $C_1$-$C_6$-Alkyl, $C_3$-$C_8$-Cycloalkyl, $C_3$-$C_8$-Cycloalkenyl, Benzyl oder Phenyl steht und
- $R^6$ und $R^7$ unabhängig voneinader jeweils für Wasserstoff, $R^5$ oder die Gruppe $-CH_2-R^3$ stehen, oder zusammen mit dem Stickstoffatom einen gesättigten, oder teilweise ungesättigten, gegebenenfalls durch ein weiteres Stickstoff-, Schwefel- und/oder Sauerstoffatom unterbrochenen 5 bis 7-gliedrigen Ring bilden, der gegebenenfalls einfach oder zweifach durch

Alkylgruppen substituiert ist.

Besonders bevorzugt werden die Verbindungen der Formel (I) verwendet, in welcher

$R^1$ und $R^2$ unabhängig voneinander für Wasserstoff, Fluor, Chlor, Brom, Methyl, Ethyl, Methoxy, Ethoxy, Trifluormethyl oder Trifluormethoxy stehen,

$R^3$ für Wasserstoff, Methyl, Ethyl, n- und i-Propyl, n-, i-, s- und t-Butyl und gegebenenfalls substituiertes Cyclopentyl, Cyclobutyl, Cyclohexyl, Cycloheptyl, Cyclopropyl, Phenyl, Naphthyl, Benzyl, Pyridyl, Pyrimidyl, Thienyl, Furfuryl oder Pyrryl steht und

$R^4$ für die Gruppe $SR^5$, $NR^6R^7$ oder $OR^5$ steht, wobei

$R^5$ für Methyl, Ethyl, n- und i-Propyl, n-, i-, s- und t-Butyl, Cyclopropyl, Cyclobutyl, Cyclopentyl, Cyclohexyl oder Phenyl steht und

$R^6$ und $R^7$ unabhängig voneinander jeweils für Wasserstoff, $R^5$ oder die Gruppe $-CH_2-R^3$ stehen oder zusammen mit den Stickstoffatomen einen Pyrrolidin-, Pyrrolin-, Oxazolidin-, Oxazolin-, Isooxazolidin-, Isooxazolin-, Pyrazolin, Pyrazolidin-, Imidazolodin-, Imidazolin-, Triazolidin-, Triazolin-, Piperidin-, Morpholin-, Thiomorpholin, N-Methylpiperazin, Piperazin-Ring bilden der gegebenenfalls durch ein oder zwei Methylgruppen substituiert ist.

Bevorzugt erfindungsgemäß zu verwendende Verbindungen sind auch Additionsprodukte aus Säuren.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Trifluoressigsäure, Maleinsäure, Bernsteinsäure, Fumarsäure, Weinsäure, Zitronensäure, Salicylsäure, Sorbinsäure und Milchsäure, Ölsäure, Stearinsäure, gegebenenfalls einfach bis mehrfach durch Nitro oder Halogen substituierte Benzoesäure, Gluconsäure, Ascorbinsäure, Äpfelsäure, Sulfamidsäure, Sulfonsäuren, wie z.B. p-Toluolsulfonsäure, 1,5-Naphthalindisulfonsäure und Methansulfonsäure, sowie Imide, wie z.B. Phthalimid, Saccharin und Thiosaccharin.

Außerdem bevorzugt erfindungsgemäß zu verwendende Verbindungen sind auch Additionsprodukte aus Salzen von Metallen der I., II. und III. Hauptgruppe sowie des Zinns, sowie ferner Salze von Metallen der I., II., VII, und VIII. Nebengruppe des Periodensystems der Elemente.

Hierbei sind Salze des Kupfers, Zinks, Mangans, Magnesiums, Calciums, Zinns, Eisens, Cobalts und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Gegenstand der Anmeldung sind desweiteren neue Thiosemicarbazide der Formel (Ia)

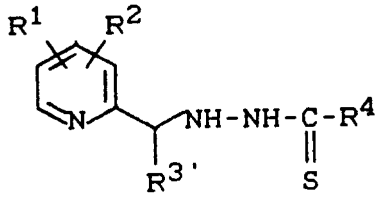

(Ia)

in welcher

$R^1$, $R^2$, $R^4$ die oben angegebene Bedeutung haben und

$R^{3'}$ für Cycloalkyl, Aryl, Aralkyl, Hetaryl oder Hetaralkyl steht,

sowie deren Metallsalz-Komplexe und Säureadditions-Salze.

In bevorzugten Verbindungen der Formel (Ia) haben $R^1$, $R^2$, $R^4$ und die entsprechenden Reste von $R^{3'}$ die schon im Zusammenhang mit der Beschreibung der erfindungsgemäßen Verwendung der Stoffe der Formel (I) angegebenen Bedeutungen und Kombinationen.

Die erfindungsgemäßen Verbindungen der Formel (Ia) werden erhalten, indem man Hydrazone der Formel (II)

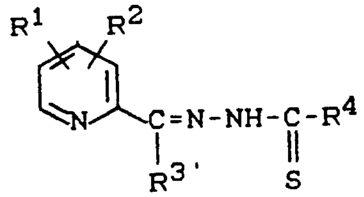

(II)

in welcher

$R^1$, $R^2$, $R^{3'}$ und $R^4$ die oben angegebene Bedeutung haben in Gegenwart von Lösungsmittel mit Natriumborhydrid reduziert.

Die Hydrazone der Formel (II) sind aus der WO-8 500 955, DE 1 934 809 und einer parallelen Anmeldung bekannt und können nach den dort angegebenen Methoden erhalten werden.

Als Lösungsmittel für die Reaktion kommen polare organische Lösungsmittel oder Lösungsmittelgemische gegebenenfalls in Mischung mit Wasser in Frage. Vorzugsweise und beispielhaft seien genannt: Alkohole wie Methanol, Ethanol, n- oder iso-Propanol und Butanol.

Die Reduktion wird bei 0 bis 80 °C vorzugsweise bei 10 bis 50 °C durchgeführt, dabei werden pro Mol der Verbindung der Formel (I) 1 bis 8, vorzugsweise 1 bis 4 Mol Natriumborhydrid eingesetzt.

Desweiteren können die Verbindungen der Formel (Ia) in welcher $R^4$ für die Gruppe $NR^5R^6$, $SR^5$ oder $OR^5$ steht dadurch hergestellt werden, daß man die entsprechenden Verbindungen der Formel (Ia) in welcher $R^4$ für die Gruppe S Me steht, mit Aminen, Mercaptanen oder Alkoholen einer Substitution unter Freisetzung von Methylmercaptan unterwirft.

Die Verbindungen der Formel (Ia) werden als antimikrobielle Mittel sowohl zur Bekämpfung von Pflanzenschädlingen als auch zum Schutz technischer Materialien erfindungsgemäß verwendet. Es ist dabei überraschend, daß die erfindungsgemäßen Verbindungen der Formel (Ia) eine erheblich bessere fungizide Wirksamkeit im Pflanzenschutz zeigen als die aus dem Stand der Technik bekannten Thiosemicarbazide.

Die Wirkstoffe der Formel (I) und (Ia) beziehungsweise die diese Wirkstoffe enthaltenden Mittel weisen eine starke Wirkung gegen Mikroorganismen auf. Sie werden daher erfindungsgemäß im Materialschutz zum Schutz technischer Materialien verwendet: sie sind vor allem wirksam gegen Schimmelpilze, holzverfärbende und holzzerstörende Pilze und Bakterien, sowie gegen Hefen, Algen und Schleimorganismen. Beispielhaft - ohne jedoch zu limitieren - seien die folgenden Gattungen von Mikroorganismen genannt:

Alternaria wie Alternaria tenuis, Aspergillus wie Aspergillus niger und Aspergillus terreus, Aureobasidium wie Aureobasidium pullulans, Chaetomium wie Chaetomium globosum, Cladosporium wie Cladosporium herbarum, Coniophora wie Coniophora puteana, Gliocladium wie Gliocladium virens, Lentinus wie Lentinus tigrinus, Paecilomyces wie Paecilomyces variotii, Penicillium wie Penicillium brevicaule, Penicillium glaucum und Penicillium pinophilum, Polyporus wie Polyporus versicolor, Sclerophoma wie Sclerophoma pityophila, Streptoverticillium wie Streptoverticillium reticulum, Trichoderma wie Trichoderma viride, Trichophyton wie Trichophyton mentagrophytes;

Escherichia wie Escherichia coli, Pseudomonas wie Pseudomonas aeruginosa, Staphylococcus wie Staphylococcus aureus;

Candida wie Candida albicans.

Die Menge der eingesetzten Wirkstoffe ist von der Art und dem Vorkommen der Mikroorganismen der Keimzahl und von dem Medium abhängig. Die optimale Einsatzmenge kann bei der Anwendung jeweils durch Testreihen ermittelt werden. Im allgemeinen ist es jedoch ausreichend 0,001 bis 20 Gew.-%, vorzugsweise 0,05 bis 10 Gew.-%, der Wirkstoffgemische, bezogen auf das zu schützende Material, einzusetzen.

Die neuen Wirkstoffe können als solche, in Form von Konzentrationen oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit Lösungs- beziehungsweise Verdünnungsmitteln, Emulgatoren, Dispergatoren und/oder Binde- oder Fixiermitteln, gegebenenfalls Sikkative und UV-Stabilisatoren und gegebenenfalls Farbstoffen und Pigmenten sowie weiteren Verarbeitungshilfsmitteln.

Als Lösungs- beziehungsweise Verdünnungsmittel kommen organisch-chemische Lösungsmittel oder Lösungsmittelgemische und/oder ein öliges beziehungsweise ölartiges organisch-chemisches Lösungsmittel oder Lösungsmittelgemisch und/oder Wasser mit vorzugsweise einem Emulgator und/Netzmittel in Frage.

Als übliche schwerflüchtige wasserunlösliche ölige oder ölhaltige Lösungsmittel werden vorzugsweise die jeweiligen Mineralöle/mineralölhaltige Lösungsmittelgemische oder deren Aromatenfraktionen verwendet. Beispielhaft seien Testbenzin, Petroleum oder Alkylbenzole genannt, daneben Spindelöl und Monochlornaphthalin. Die Siedebereiche dieser schwerflüchtigen Lösemittel(gemische) überstreichen den Bereich von ca. 170 °C bis maximal 350 °C.

Die vorbeschriebenen schwerflüchtigen öligen oder ölartigen Lösungsmittel können teilweise oder ganz durch leichter flüchtige organisch-chemische Lösungsmittel ersetzt werden.

Zur Herstellung eines Holzschutzmittels wird vorzugsweise ein Teil des oben beschriebenen Lösungsmittels oder Lösungsmittelgemisches durch ein polares organischchemisches Lösungsmittel oder Lösungsmittelgemisches ersetzt. Vorzugsweise gelangen dabei Lösungsmittel, die Hydroxygruppen, Estergruppen, Ethergruppen oder Gemische dieser Funktionalität enthalten, zum Einsatz. Beispielhaft seien Ester oder Glykolether genannt. Als Bindemittel werden erfindungsgemäß verstanden wasserverdünnbare beziehungsweise in organisch-chemischen Lösungsmitteln löslich, dispergier- oder emulgierbare Kunstharze, bindende trocknende Öle, z.B. auf Basis von Acrylharzen, Vinylharzen, Polyesterharzen, Polyurethanharzen, Alkydharzen, Phenolharzen, Kohlenwasserstoffharzen, Silikonharzen. Das benutzte Bindemittel kann als Lösung, Emulsion oder Dispersion eingesetzt werden. Vorzugsweise werden Gemische aus Alkydharzen und trocknendem pflanzlichen Öl verwendet. Besonders bevorzugt sind Alkydharze mit einem Ölanteil zwischen 45 und 70 %.

Das erwähnte Bindemittel kann ganz oder teilweise durch ein Fixierungsmittelgemisch oder ein Weichmachergemisch ersetzt werden. Diese Zusätze sollen einer Verflüchtigung der Wirkstoffe sowie einer Kristallisation beziehungsweise eines Ausfällens vorbeugen. Vorzugsweise ersetzen sie 0,01 bis 30 % des Bindemittels (bezogen auf 100 % des eingesetzten Bindemittels).

Die Weichmacher stammen aus den chemischen Klassen oder Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat und Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylbenzophenon.

Als Lösungs- beziehungsweise Verdünnungsmittel kommt vorzugsweise Wasser in Frage, gegebenenfalls in Mischung mit einem oder mehreren der obengenannten Lösungsbeziehungsweise Verdünnungsmittel, Emulgatoren und Dispergatoren.

Technische Materialien sind erfindungsgemäß nicht lebende Materialien, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Bevorzugte technische Materialien im Sinne der Erfindung sind Klebstoffe, Leime, Papiere und Kartone, Leder, Holz, Anstrichmittel, Kühlschmiermittel, wäßrige Hydraulikflüssigkeiten und Kühlkreisläufe und allgemein wäßrige funktionelle Flüssigkeiten.

Die Wirksamkeit und das Wirkungsspektrum der erfindungsgemäßen Wirkstoffe beziehungsweise den daraus herstellbaren Mitteln, Konzentraten oder ganz allgemein Formulierungen kann erhöht werden, wenn gegebenenfalls weitere antimikrobiell wirksame Verbindungen, Fungizide, Bakterizide, Herbizide, Insektizide oder andere Wirkstoffe zur Vergrößerung des Wirkungsspektrums oder Erzielung besonderer Effekte wie z.B. dem zusätzlichen Schutz vor Insekten zugesetzt werden. Diese Mischungen können ein breiteres Wirkungsspektrum besitzen als die erfindungsgemäßen Verbindungen.

In vielen Fällen erhält man dabei synergistische Effekte, das heißt, die Wirksamkeit der Mischung ist größer als die der Wirksamkeit der Einzelkomponenten. Besonders günstige Mischungspartner sind z.B. die folgenden Verbindungen:

Sulfenamide wie Dichlofluanid (Euparen), Tolylfluanid (Methyleuparen), Folpet, Fluorfolpet;

Benzimidazole wie Carbendazim (BCM), Benomyl, Fuberidazol, Thiabendazol oder deren Salze;

Thiocyanate wie Thiocyanatomethylthiobenzothiazol (TCMTB), Methylenbisthiocyanat (MBT);

quartäre Ammoniumverbindungen wie Benzyl-dimethyl-tetradecyl-ammoniumchlorid, Benzyl-dimethyl-dodecyl-ammoniumchlorid, Didecyl-dimethyl-ammoniumchlorid;

Morpholinderivate wie $C_{11}$-$C_{14}$-4-Alkyl-2,6-dimethyl-morpholin-homologe (Tridemorph), (±)-cis-4-[3-tert-Butylphenyl)-2-methylpropyl]-2,6-dimethylmorpholin (Fenpropimorph), Falimorph;

Phenole wie o-Phenylphenol, Tribromphenol, Tetrachlorphenol, Pentachlorphenol, 3-Methyl-4-chlorphenol,

Dichlorophen, Chlorophen oder deren Salze;
Azole wie Triadimefon, Triadimenol, Bitertanol, Tebuconazole, Propionazole, Azaconazole, Hexaconazole, Prochloraz;
Iodpropargylderivate wie Iodpropargyl-butylcarbamat (IPBC), -chlorophenylformal, -phenylcarbamat, -hexylcarbamat, -cyclohexylcarbamat, Iodpropargyloxyethylphenylcarbamat;
Iodderivate wie Diiodmethyl-arylsulfone z.B. Diiodmethyl-p-tolylsulfon;
Bromderivate wie Bronopol;
Isothiazolinone wie N-Methylisothiazolin-3-on, 5-Chloro-N-methylisothiazolin-3-on, 4,5-Dichloro-N-octylisothiazolin-3-on, N-Octylisothiazolin-3-on (Octhilinone);
Benzoisothiazolinone, Cyclopentenisothiazolinon;
Pyridine wie 1-Hydroxy-2-pyridinthion (und ihre Na-, Fe-, Mn, Zn-Salze), Tetrachlor-4-methylsulphonylpyridin;
Metallseifen wie Zinn-, Kupfer-, Zink-naphthenat, -octoat, -2-ethylhexanoat, -oleat, -benzoat, Oxide wie TBTO, $Cu_2O$, CuO, ZnO;
Organische Zinnverbindungen wie Tributylzinnnaphthenat und Tributylzinnoxid;
Dialkyldithiocarbamate wie Na- und Zn-Salze von Dialkyldithiocarbamaten, Tetramethylthiuramdisulfid (TMTD);
Nitrile wie 2,4,5,6-Tetrachlorisophthalonitril (Chlorthalonil) u.a. Mikrobizide mit aktivierten Halogengruppen wie Cl-Ac, MCA, Tectamer, Bronidox;
Benzthiazole wie 2-Mercaptobenzothiazol; s.o. Dazomet;
Chinoline wie 8-Hydroxychinolin;
Formaldehydabspaltende Verbindungen wie Benzylalkoholmono(poly)hemiformal, Oxazolidine, Hexahydro-s-triazine, N-Methylolchloracetamid;
Tris-N-(Cyclohexyldiazeniumdioxy)-Aluminium N-(Cyclohexyldiazeniumdioxy)-Tributylzinn beziehungsweise K-Salze, Bis-(N-cyclohexyl)diazinium (-dioxy- Kupfer oder Aluminium);
Als Insektizide werden bevorzugt zugesetzt:
Phosphorsäureester wie Azinphos-ethyl, Azinphos-methyl, 1-(4-Chlorphenyl)-4-(O-ethyl, S-propyl)-phosphoryloxypyrazol (TIA-230), Chlorpyrifos, Coumaphos, Demeton, Demeton-S-methyl, Diazinon, Dichlorvos, Dimethoate, Ethoprophos, Etrimfos, Fenitrothion, Fention, Heptenophos, Parathion, Parathion-methyl, Phosalone, Phoxion, Pirimiphos-ethyl, Pirimiphos-methyl, Profenofos, Prothiofos, Sulprofos, Triazophos und Trichlorphon.

Carbamate wie Aldicarb, Bendiocarb, BPMC (2-(1-Methylpropyl)phenylmethylcarbamat), Butocarboxim, Butoxycarboxim, Carbaryl, Carbofuran, Carbosulfan, Cloethocarb, Isoprocarb, Methomyl, Oxamyl, Pirimicarb, Promecarb, Propoxur und Thiodicarb.

Pyrethroide wie Allethrin, Alphamethrin, Bioresmethrin, Byfenthrin (FMC 53 800), Cycloprothrin, Cyfluthrin, Decamethrion, Cyhalothrin, Cypermethrin, Deltamethrin, Alpha-cyano-3-phenyl-2-methylbenzyl-2,2-dimethyl-3-(2-chlor-2-trifluormethylvinyl)cyclopropancarboxylat, Fenpropathrin, Fenfluthrin, Fenvalerate, Flucythrinate, Flumethrin, Fluvalinate, Permethrin und Resmethrin; Nitroimino und Nitroimide wie 1-[(6-Chlor-3-pyridinyl)-methyl]-4,5-dihydro-N-nitro-1H-imidazol-2-anin (Imidacloprid).

Organosiliciumverbindungen, vorzugsweise Dimethyl(phenyl)silylmethyl-3-phenoxy-benzylether wie z.B. Dimethyl(4-ethoxyphenyl)-silylmethyl-3-phenoxy-benzylether oder Dimethyl(phenyl)-silylmethyl-2-phenoxy-6-pyridylmethylether wie z.B. Dimethyl(9-ethoxyphenyl)-silylmethyl-2-phenoxy-6-pyridylmethylether oder (Phenyl)[3-phenoxyphenyl)-propyl](dimethyl)-silane wie z.B. (4-ethoxy-phenyl)-[3-(4-fluoro-3-phenoxyphenyl)-propyl]dimethyl-silan.

Als andere Wirkstoffe kommen in Betracht Algizide, Molluskizide, Wirkstoffe gegen "sea animals", die sich auf z.B. Schiffsbodenanstrichen ansiedeln.

Die zum Schutz der technischen Materialien verwendeten mikrobiziden Mittel der Konzentrate enthalten die erfindungsgemäßen Wirkstoffe in einer Konzentration von 0,01 bis 95 Gew.-%, insbesondere 0,01 bis 60 Gew.-%, daneben gegebenenfalls 0,001 bis 10 Gew.-% eines geeigneten weiteren Fungizids, Insektizids oder eines weiteren Wirkstoffs wie oben genannt.

Die erfindungsgemäßen Wirkstoffe beziehungsweise Mittel ermöglichen in vorteilhafter Weise, die bisher verfügbaren mikrobiziden Mittel durch effektivere zu ersetzen. Sie zeigen eine gute Stabilität und haben in vorteilhafter Weise ein breites Wirkungsspektrum.

Desweiteren weisen die erfindungsgemäßen Wirkstoffe der Formel (Ia) eine starke Wirkung gegen Pflanzen-Schädlinge auf und können zur Bekämpfung von unerwünschten Schadorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Phythophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder Peronospora brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder Pyrenophora graminea (Konidienform: Drechslera, Synonym: Helminthosporium);
Cochliobolus-Arten, wie beispielsweise Cochliobolus sativus (Konidienform: Drechslera, Synonym: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethyl sulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Die nachfolgenden Beispiele dienen zur Erläuterung der Erfindung ohne sie darauf zu limitieren. Teile und Prozentangaben bedeuten Gewichtsteile beziehungsweise Gewichtsprozente.

Beispiel 1

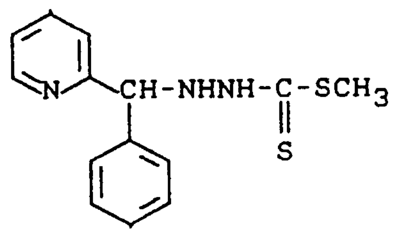

In 100 ml Ethanol werden 15,94 g (0,056 mol) 1-(Methylthio-thiocarbonyl)-2-pyridylbenzoyl-hydrazon vorgelegt und dann 2,5 g (0,066 mol) Natriumborhydrid portionsweise innerhalb 90 min bei Raumtemperatur zugesetzt. Nach Zugabe von 50 ml $H_2O$ wird mit Essigsäure neutral gestellt, abgesaugt und getrocknet. Da die Umsetzung noch nicht vollständig ist, wird erneut in 70 ml Ethanol aufgenommen und mit 4,0 g (0,106 mol) $NaBH_4$ portionsweise reduziert. Nach gleicher Aufarbeitung ergeben sich 9,93 g (62 %) 1-(Methylthio-thiocarbonyl)-2-pyridylbenzoyl-hydrazin vom Schmelzpunkt 120 bis 144 °C.

Beispiel 7

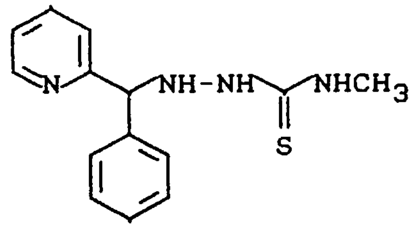

7,23 g (0,025 mol) 1-(Methylthio-thiocarbonyl)-2-pyridylbenzoyl-hydrazin werden in 80 ml Ethanol 5 h refluriert, wobei die Atmosphäre im Reaktionsgefäß mehrfach mit Methylamin gesättigt wird. Nach Abkühlen werden 3,49 g (64 %) 1-(N-Methylamino-thiocarbonyl)-2-(pyridylbenzoyl)-hydrazin) vom Schmelzpunkt 150 bis 152 °C erhalten.

In analoger Weise zu der in den Beispielen 1 und 7 beschriebenen Methoden und unter Berücksichtigung der Angaben in der Beschreibung, werden die in der nachfolgenden Tabelle aufgeführten Verbindungen der Formel (I)'

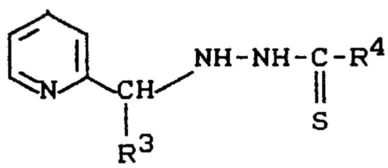

hergestellt:

| Nr. | $R^3$ | $R^4$ | F[°C] |
|---|---|---|---|
| 1 | H | $-SCH_3$ | 121 |
| 2 | $-CH_3$ | " | 112 |
| 3 | -Phenyl | " | 120-44 |
| 4 | -2-Pyridyl | " | 121 |
| 5 | H | $-NHCH_3$ | - |
| 6 | $-CH_3$ | " | - |
| 7 | -Phenyl | " | 150-2 |
| 8 | -2-Pyridyl | " | - |
| 9 | H | $-N(CH_3)_2$ | - |
| 10 | $-CH_3$ | " | - |
| 11 | -Phenyl | " | 169 |
| 12 | -2-Pyridyl | " | - |
| 13 | H | $-N(C_2H_5)_2$ | - |
| 14 | $-CH_3$ | " | - (Produkt Semikristallin) |
| 15 | -Phenyl | " | 145-6 |
| 16 | -2-Pyridyl | " | - |
| 17 | H | $-N(n-C_3H_7)_2$ | - |
| 18 | $-CH_3$ | " | ≤ 30 (Semikristallin) |
| 19 | -Phenyl | " | 114-6 |
| 20 | -2-Pyridyl | " | - |
| 21 | H | $-N(n-C_4H_9)_2$ | - |
| 22 | $-CH_3$ | " | ≤ 30 (Semikristallin) |
| 23 | -Phenyl | " | 94-9 |
| 24 | -2-Pyridyl | " | - |
| 25 | H | $-N(n-C_5H_{11})_2$ | - |
| 26 | $-CH_3$ | " | - |
| 27 | -Phenyl | " | 81-3 |
| 28 | -2-Pyridyl | " | - |
| 29 | H | $-N(n-C_6H_{13})_2$ | - |
| 30 | $-CH_3$ | " | - |
| 31 | -Phenyl | " | 48-68 |
| 32 | -2-Pyridyl | " | - |

| Nr. | $R^3$ | $R^4$ | F[° C] |
|---|---|---|---|
| 33 | H | 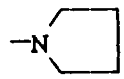 | - |
| 34 | $-CH_3$ | | - |
| 35 | -Phenyl | | 189 |
| 36 | -2-Pyridyl | | - |
| 37 | H | 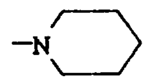 | 153 |
| 38 | $-CH_3$ | | 164 |
| 39 | -Phenyl | | 41 |
| 40 | -2-Pyridyl | | - |
| 41 | H | 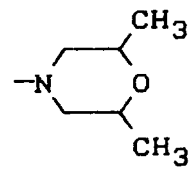 | - |
| 42 | $-CH_3$ | | 169 |
| 43 | -Phenyl | | 154 |
| 44 | -2-Pyridyl | | - |
| 45 | H | 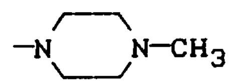 | - |
| 46 | $-CH_3$ | | 146 |
| 47 | -Phenyl | | - |
| 48 | -2-Pyridyl | | - |
| 49 | H | 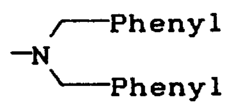 | - |
| 50 | $-CH_3$ | | 141 |
| 51 | -Phenyl | | - |
| 52 | -2-Pyridyl | | - |
| 53 | H | 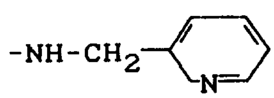 | - |
| 54 | $-CH_3$ | | 110-17 |
| 55 | -Phenyl | | - |
| 56 | -2-Pyridyl | | - |
| 57 | H | 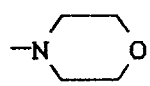 | - |
| 58 | $-CH_3$ | | - |
| 59 | -Phenyl | | - |
| 60 | -2-Pyridyl | | - |

Anwendungsbeispiel

A. Zum Nachweis der Wirksamkeit gegen Pilze werden die minimalen Hemm-Konzentrationen (MHK) von erfindungsgemäßen Mitteln bestimmt:
Ein Agar, der unter Verwendung von Malzextrakt hergestellt wird, wird mit erfindungemäßen Wirkstoffen in Konzentrationen von 0,1 mg/l bis 5.000 mg/l versetzt. Nach Erstarren des Agars erfolgt Kontamination mit Reinkulturen der in der Tabelle 1 aufgeführten Testorganismen. Nach 2-wöchiger Lagerung bei 28 °C und 60 bis 70 % relativer Luftfeuchtigkeit wird die MHK bestimmt. MHK ist die niedrigste Konzentration an Wirkstoff, bei der keinerlei Bewuchs durch die verwendete Mikrobenart erfolgt, sie ist in der nachstehenden Tabelle 2 angegeben.

Tabelle 2 (Vergleich)

MHK's [mg/l] bei der Einwirkung erfindungsgemäßer Verbindungen auf Mikroorganismen

| Bsp. | Penicillium brevicaule | Chaetomium globusum | Aspergillus niger | Lentinus tigrinus | Bacillus sublilis Staphylococcus aureus | Pseudomonas aeruginosa | Sclerophoma pityophila | Trichoderma viride | Cladosporium herbarum | Alternaria tenuis | Aureobasidium pullulans |
|---|---|---|---|---|---|---|---|---|---|---|---|
| 1 | < 400 | < 400 | < 800 | - | > 800 | < 800 | < 400 | < 400 | < 400 | < 400 | < 400 |
| 2 | < 400 | < 400 | < 800 | - | < 800 | > 800 | < 400 | < 400 | < 400 | < 400 | < 400 |
| 3 | < 400 | < 400 | < 800 | <50/56 | < 800 | > 800 | < 400 | < 400 | < 400 | < 400 | < 400 |
| 4 | < 400 | < 400 | < 800 | - | < 800 | < 800 | < 400 | < 400 | < 400 | < 400 | < 400 |
| 7 | > 800 | > 800 | > 800 | - | > 800 | > 800 | < 400 | > 800 | > 800 | > 800 | > 800 |
| 11 | < 400 | < 400 | < 800 | - | > 800 | > 800 | < 400 | < 400 | < 400 | < 400 | < 400 |
| 14 | < 400 | < 400 | < 800 | - | < 800 | < 800 | < 400 | < 400 | < 400 | < 400 | < 400 |
| 15 | < 400 | < 400 | < 800 | - | < 800 | > 800 | - | - | - | - | - |
| 18 | < 400 | < 400 | < 800 | - | < 800 | < 800 | < 400 | < 400 | 400 | < 400 | < 400 |
| 19 | < | < 400 | < 800 | - | < 800 | > 800 | - | - | - | - | - |
| 22 | < 400 | < 400 | < 800 | - | < 800 | < 800 | < 400 | < 400 | < 400 | < 400 | < 400 |
| 23 | < 400 | < 400 | < 800 | - | < 800 | > 800 | - | - | - | - | - |
| 27 | < 400 | < 400 | < 800 | - | < 800 | > 800 | - | - | - | - | - |
| 31 | < 400 | < 400 | < 800 | - | < 800 | > 800 | - | - | - | - | - |
| 35 | < 400 | < 400 | < 800 | - | > 800 | > 800 | < 400 | < 400 | < 400 | < 400 | < 400 |
| 37 | < 400 | < 400 | < 800 | - | < 800 | < 800 | < 400 | < 400 | < 400 | < 400 | < 400 |
| 38 | < 400 | < 400 | < 800 | - | > 800 | > 800 | < 400 | < 400 | < 400 | < 400 | < 400 |
| 39 | < 400 | < 400 | < 800 | - | > 800 | > 800 | < 400 | < 400 | < 400 | < 400 | < 400 |
| 42 | < 400 | < 400 | < 800 | - | < 800 | < 800 | < 400 | < 400 | < 400 | < 400 | < 400 |
| 43 | < 400 | < 400 | < 800 | - | > 800 | > 800 | < 400 | < 400 | < 400 | < 400 | < 400 |
| 46 | < 400 | < 400 | < 800 | - | < 800 | < 800 | < 400 | < 400 | < 400 | < 400 | < 400 |
| 50 | < 400 | < 400 | > 800 | - | > 800 | > 800 | < 400 | < 400 | < 400 | < 400 | < 400 |
| 54 | > 800 | > 800 | > 800 | - | 800 | > 800 | - | - | - | - | - |

Beispiel B

Botrytis-Test (Buschbohne) / protektiv

Lösungsmittel: 4,7 Gewichtsteile Aceton

Emulgator: 0,3 Gewichtsteile Alkyl-Aryl-Polyglykolether

Zur Herstellung einer zweckmäßigen Wirkstoffzubereitung vermischt man 1 Gewichtsteil mit den angegebnen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea

bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

So zeigen z.B. die Verbindungen 11, 15, 23, 27, 35 und 43 bei einer Wirkstoffkonzentration von 100 ppm einen Wirkungsgrad von 92 bis 100 %.

Beispiel C

Phaedon-Larven-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil mit den angegebnen Mengen Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

Bei diesem Test zeigen z.B. die Verbindungen der Herstellungsbeispiele 4, 7, 14, 43, 58 und 59 bei einer Wirkstoffkonzentration von 0,1 % nach 7 Tagen einen 100%igen Abtötungsgrad.

Beispiel D

Plutella-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil mit den angegebnen Mengen Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

Bei diesem Test zeigen z.B. die Verbindungen der Herstellungsbeispiele 14, 19 und 58 bei einer Wirkstoffkonzentration von 0,1 % nach 7 Tagen einen 100%igen Abtötungsgrad.

**Patentansprüche**

**1.** Verwendung der Verbindungen der Formel (I)

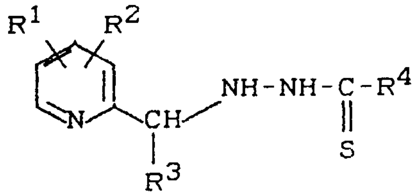

(I)

in welcher

| | |
|---|---|
| $R^1$ und $R^2$ | unabhängig voneinander jeweils für Wasserstoff, Alkyl, Alkoxy, Thioalkyl, Halogen, Halogenalkyl, Halogenalkoxy oder Halogenthioalkyl stehen oder zusammen einen Benzol- oder Cycloalkylring bilden, |
| $R^3$ | für Wasserstoff, Alkyl, Cycloalkyl, Aryl, Aralkyl, Hetaryl oder Hetaralkyl steht und |
| $R^4$ | für die Gruppen $SR^5$, $NR^6R^7$ oder $OR^5$ steht, wobei |
| $R^5$ | für Alkyl, Cycloalkyl, Cycloalkenyl, Aralkyl oder Aryl steht und |

$R^6$ und $R^7$ unabhängig voneinander jeweils für Wasserstoff, $R^5$ oder die Gruppe $-CH_2-R^3$ stehen, oder zusammen mit dem Stickstoffatom einen gesättigten bis ungesättigten, gegebenenfalls substituierten Heterocyclus bilden,

sowie deren Metallsalzkomplexe und Säureadditions-Salze, zum Schutz von technischen Materialien.

**2.** Verwendung der Verbindungen der Formel (I) gemäß Anspruch 1, in welcher

$R^1$ und $R^2$ unabhängig voneinander für Wasserstoff, Halogen, $C_1$-$C_6$-Alkyl, -Alkoxy, -Thioalkyl, -Halogenalkyl, -Halogenalkoxy oder -Halogenthioalkyl stehen oder zusammen einem Benzol-, Cyclopenton- oder Cyclohexanring bilden

$R^3$ für Wasserstoff, $C_1$-$C_{10}$-Alkyl, $C_3$-$C_8$-Cycloalkyl, Phenyl, Naphthyl, Benzyl, Pyridyl, Pyrimidyl, Thienyl, Furfuryl oder Pyrryl steht und

$R^4$ für die Gruppe $SR^5$, $NR^6R^7$ oder $OR^5$ steht, wobei

$R^5$ für $C_1$-$C_6$-Alkyl, $C_3$-$C_8$-Cycloalkyl, $C_3$-$C_8$-Cycloalkenyl, Benzyl oder Phenyl steht und

$R^6$ und $R^7$ unabhängig voneinader jeweils für Wasserstoff, $R^5$ oder die Gruppe $-CH_2-R^3$ stehen, oder zusammen mit dem Stickstoffatom einen gesättigten, teilweise ungesättigten, gegebenenfalls durch ein weiteres Stickstoff-, Schwefel- und/oder Sauerstoffatom unterbrochenen 5 bis 7-gliedrigen Ring bilden, der gegebenenfalls einfach oder zweifach durch Alkylgruppen substituiert ist.

**3.** Verwendung der Verbindungen der Formel (I) gemäß Anspruch 1, in welcher

$R^1$ und $R^2$ unabhängig voneinander für Wasserstoff, Fluor, Chlor, Brom, Methyl, Ethyl, Methoxy, Ethoxy, Trifluormethyl oder Trifluormethoxy stehen,

$R^3$ für Wasserstoff, Methyl, Ethyl, n- und i-Propyl, n-, i-, s- und t-Butyl und gegebenenfalls substituiertes Cyclopentyl, Cyclobutyl, Cyclohexyl, Cycloheptyl, Cyclopropyl, Phenyl, Naphthyl, Benzyl, Pyridyl, Pyrimidyl, Thienyl, Furfuryl oder Pyrryl steht und

$R^4$ für die Gruppe $SR^5$, $NR^6R^7$ oder $OR^5$ steht, wobei

$R^5$ für Methyl, Ethyl, n- und i-Propyl, n-, i-, s- und t-Butyl, Cyclopropyl, Cyclobutyl, Cyclopentyl, Cyclohexyl, Benzyl oder Phenyl steht und

$R^6$ und $R^7$ unabhängig voneinander jeweils für Wasserstoff, $R^5$ oder die Gruppe $-CH_2-R^3$ stehen oder zusammen mit den Stickstoffatomen einen Pyrrolidin-, Pyrrolin-, Oxazolidin-, Oxazolin-, Isooxazolidin-, Isooxazolin-, Pyrazolin, Pyrazolidin-, Imidazolodin-, Imidazolin-, Triazolidin-, Triazolin-, Piperidin-, Morpholin-, Thiomorpholin, N-Methylpiperazin, Piperazin-Ring bilden der gegebenenfalls durch ein oder zwei Methylgruppen substituiert ist.

**4.** Thiosemicarbazide der Formel (Ia)

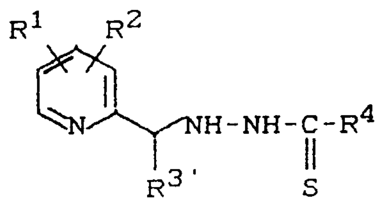

(Ia)

in welcher

$R^1$, $R^2$, $R^4$ die in den Ansprüchen 1 bis 3 angegebenen Bedeutungen haben und

$R^{3'}$ für Cycloalkyl, Aryl, Aralkyl, Hetaryl oder Hetaralkyl steht,

sowie deren Metallsalz-Komplexe und Säureadditions-Salze,

**5.** Verfahren zur Herstellung von Verbindungen der Formel (Ia) nach Anspruch 4, dadurch gekennzeichnet, daß man Hydrazone der Formel (II)

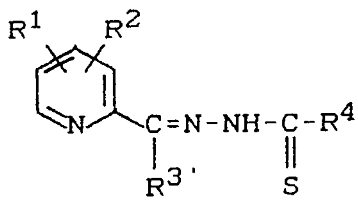

(II)

in welcher
$R^1$, $R^2$, $R^{3'}$ und $R^4$ die oben angegebene Bedeutung haben in Gegenwart von Lösungsmittel mit Natriumborhydrid reduziert.

**6.** Schädlingsbekämpfungsmittel, gekennzeichnet durch einen Gehalt an mindestens einem Thiosemicarbazid der Formel (Ia) nach Anspruch 4.

**7.** Verwendung von Thiosemicarbaziden der Formel (I) oder (Ia) nach Anspruch 1 oder 4 zur Bekämpfung von Schädlingen.

**8.** Verfahren zur Bekämpfung von Schädlingen, dadurch gekennzeichnet, daß man Thiosemicarbazide der Formel (Ia) nach Anspruch 4 auf Schädlinge und/oder ihren Lebensraum einwirken läßt.

**9.** Verfahren zur Herstellung von Schädlingsbekämpfungsmitteln, dadurch gekennzeichnet, daß man Thiosemicarbazide der Formel (Ia) nach Anspruch 4 mit Streckmitteln und/oder oberflächenaktiven Mitteln vermischt.

**10.** Antimikrobielle Mittel zum Schutz von technischen Materialien, gekennzeichnet durch einen Gehalt an mindestens einem Thiosemicarbazid der Formel (I) oder (Ia) nach Anspruch 1 bis 4.

Europäisches Patentamt

# EUROPÄISCHER RECHERCHENBERICHT

Nummer der Anmeldung
EP 93 10 7998

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,X<br>D,Y | WO-A-8 504 333 (A/S CHEMINOVA)<br>10. Oktober 1985<br>--- | 1-3,7,10<br>1-10 | C07D213/42<br>C07D295/195<br>C07D413/12<br>A01N47/24<br>A01N47/34 |
| X<br>Y | PESTIC. SCI.<br>Bd. 30, 1990,<br>Seiten 223 - 233 'Chitin Biosynthesis Inhibition and fungicidal effect of thiosemicarbazones of 2-formyl and 2-acetylpyridine, their hydrogenated derivatives and copper complexes thereof.'<br>--- | 1-3,7,10<br>1-10 | |
| X<br>Y | EXPERIENTIA<br>Bd. 45, Nr. 6, 1989,<br>Seiten 580 - 583<br>'Arylpyridyl-thiosemicarbazones: A new class of anti-juvenile hormones active against Lepidoptera'<br>--- | 1-3,7,10<br>1-10 | |
| A | ANTIVIRAL RESEARCH<br>Bd. 6, Nr. 4, 1986,<br>Seiten 197 - 222 'Thiosemicarbazones of 2-acetylpyridine, 2-acetylquinoline, 1-acetylisoquinoline and related compounds as inhibitors of herpes simplex virus in vitro and in a cutaneous herpes guinea pig model.'<br>--- | 1-10 | RECHERCHIERTE SACHGEBIETE (Int. Cl.5)<br>C07D<br>A01N |
| Y | JOURNAL OF AGRICULTURAL AND FOOD CHEMISTRY<br>Bd. 31, Nr. 4, 1983,<br>Seiten 713 - 718 '2-Acetylpyridine Thiosemicarbazones as inhibitors of Ecdysis in Oncopeltus fasciatus: Structure-Activity Relationship study'<br>----- | 1-10 | |

Der vorliegende Recherchenbericht wurde für alle Patentansprüche erstellt

| Recherchenort | Abschlußdatum der Recherche | Prüfer |
|---|---|---|
| MUENCHEN | 13 SEPTEMBER 1993 | SCRUTON-EVANS I. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)